Europäisches Patentamt

European Patent Office

Office européen des brevets

(11) Publication number: **0 456 839 B1**

# EUROPEAN PATENT SPECIFICATION

(45) Date of publication of patent specification: **31.08.94**   (51) Int. Cl.⁵: **C07C 15/14**, C07C 2/66

(21) Application number: **91900341.8**

(22) Date of filing: **05.12.90**

(86) International application number:
**PCT/JP90/01577**

(87) International publication number:
**WO 91/08181 (13.06.91 91/13)**

(54) **PROCESS FOR PRODUCING 4,4'-DIISOPROPYLBIPHENYL.**

(30) Priority: **05.12.89 JP 316813/89**

(43) Date of publication of application:
**21.11.91 Bulletin 91/47**

(45) Publication of the grant of the patent:
**31.08.94 Bulletin 94/35**

(84) Designated Contracting States:
**DE FR GB**

(56) References cited:

**No relevant documents disclosed**

(73) Proprietor: **OSAKA GAS COMPANY LIMITED**
**1-2 Hiranomachi 4-chome,**
**Chuo-ku**
**Osaka-shi, Osaka 541 (JP)**

(72) Inventor: **MATSUMOTO, Masaru**
**1-22-21, Masago**
**Ibaraki-shi**
**Osaka 567 (JP)**
Inventor: **SUDA, Yasuhiro**

**4-8-A-514, Kamishinden**
**Toyonaka-shi**
**Osaka 565 (JP)**
Inventor: **YUZU, Satoshi**
**6-3-4-1303, Torishima**
**Konohana-ku**
**Osaka-shi Osaka 554 (JP)**
Inventor: **KAKIHARA, Naomichi**
**8-13-403, Kumanocho**
**Nishinomiya-shi**
**Hyogo 663 (JP)**

(74) Representative: **Patentanwälte Beetz - Timpe -**
**Siegfried Schmitt-Fumian - Mayr**
**Steinsdorfstrasse 10**
**D-80538 München (DE)**

## Description

The present invention relates to a process for producing 4,4'-diisopropylbiphenyl.

The values of "%" and "ppm" used herein are all by weight unless otherwise specified.

Prior Art

Biphenyl compounds having alkyl radicals are useful as starting materials for polymers, intermediates for drugs and agrochemicals, starting materials for liquid crystal polymers and the like. Biphenyl compounds substituted by alkyl radicals at the 4- and 4'-positions, in particular, are very important, in terms of the value added, as starting materials for heat-resistant polymers and liquid crystal polymers, for instance.

Japanese Kokai Patent Applications No. 63-122636 and No. 1-165531 disclose that a mordenite type zeolite catalyst is excellent in the selectivity for alkylation of the p-positions of biphenyl. In the processes described in the above patent publications, biphenyl synthesized by dimerizing benzene of low impurity is used as the starting material and, therefore, the total nitrogen content of the biphenyl is as low as 5 ppm or less. However, the total amount of sulfur in commercially available high-purity biphenyl is 5 ppm at the minimum and may at times amounts to as high as 50 ppm. Since the sulfur compounds contained as impurities are aromatic compounds having sulfur-containing heterocyclic structures such as benzothiophenes and the like which are close in the boiling point to biphenyl, it is practically impossible to remove these sulfur compounds to further purify the biphenyl. While the processes disclosed in the patent publications referred to above employ a batch reaction, the experimental study conducted by the inventors revealed that, in such a reaction system, the sulfur-containing impurities are adsorbed on the catalyst. In other words, in order that the reaction may be conducted in a continuous mode on a commercial scale, the starting biphenyl must be thoroughly desulfurized beforehand to insure a practically useful catalyst life. Furthermore, when the processes described in these patent publications are executed continuously, sulfur in the starting biphenyl deposits on the catalyst to cause rapid deactivation of the catalyst.

It is foreseen that an increasing proportion of biphenyl for future industrial use will be supplied by the biphenyl produced by distillation of tar but such biphenyl of tar origin is rich in sulfur compounds such as benzothiophenes and the like. However, it is very difficult to purify such tar-based biphenyl to a sufficient degree and it is impossible to utilize such biphenyl as an industrial starting material for the production of 4,4'-diisopropylbiphenyl.

Means for Solving the Problems

The inventors conducted extensive research in view of the serious problem of catalyst poisoning which is inevitable with the conventional method for producing 4,4'-diisopropylbiphenyl from biphenyl and found that diisopropylbiphenyl can be produced with high para-position selectivity and in good yield, with inhibition of catalyst poisoning, when the reaction is conducted in the presence of water using biphenyl and propylene as starting materials and a high-silica hydrated H-mordenite type zeolite having a $SiO_2/Al_2O_3$ molar ratio within a certain range or a corresponding Pt-substituted mordenite type zeolite catalyst as the catalyst.

The present invention, thus, provides the following production process:

A process for producing 4,4'-diisopropylbiphenyl comprising reacting biphenyl having a sulfur content of not more than 1% or a mixture of biphenyl and isopropylbiphenyl having a sulfur content of not more than 1% with propylene in the presence of a hydrated H-mordenite type zeolite catalyst having a $SiO_2/Al_2O_3$ molar ratio of not less than 15 or a corresponding hydrated Pt-mordenite type zeolite catalyst having a $SiO_2/Al_2O_3$ molar ratio of not less than 15 and in the presence of water.

The starting material to be used in the present invention is biphenyl or a mixture of biphenyl and isopropylbiphenyl. The latter mixture is generally an unreacted fraction separated from the reaction mixture by distillation. The total amount of sulfur compounds in the starting material may be 1% at the maximum but is preferably not more than 5000 ppm.

The isopropylating agent is propylene in the process of the present invention.

In the process according to the invention, it is essential to employ a hydrated mordenite type zeolite catalyst having a $SiO_2/Al_2O_3$ molar ratio of not less than 15. There is no upper limit to $SiO_2/Al_2O_3$ molar ratio but it is preferably about 250. The catalyst is not limited to a hydrated H-mordenite type zeolite catalyst but may be a hydrated Pt-mordenite type zeolite catalyst obtained by ion exchange of the former with Pt ions. The hydrated Pt-mordenite type zeolite is preferred because it can be regenerated at a lower temperature (lower than about 100 °C).

2

EP 0 456 839 B1

In the process according to the invention, it is essential to conduct the reaction in the presence of water. If the reaction is carried out in the absence of water, the desired effect will not be attained. This is presumably because, in the absence of water, the water of hydration is lost from the hydrated catalyst to thereby alter the structure or characteristics of the catalyst.

It is not essential to use a solvent for dilution in conducting the reaction but there may be employed such solvents as saturated straight chain hydrocarbons such as n-decane, n-undecane, n-dodecane, etc., saturated alicyclic hydrocarbons such as decalin, bicyclohexyl and so on.

In the process according to the present invention, either biphenyl or a mixture of biphenyl and isopropyl-biphenyl is reacted with propylene in the presence of a hydrated H-mordenite type zeolite catalyst having a $SiO_2/Al_2O_3$ molar ratio of not less than 15 or the corresponding Pt-mordenite type zeolite catalyst in the presence of water, and either in the presence of a solvent or in the absence thereof. The reaction conditions are not critical but the reaction is usually conducted using about 1 to 20 moles of propylene per mole of biphenyl or a mixture of biphenyl and isopropylbiphenyl and about 1 to 10 parts of water as an additive per one part of catalyst at a temperature of about 150 to 350°C (preferably about 175 to 250°C) and a pressure of atmospheric pressure to about 9.8 Mpa (100 kg/cm$^2$) (preferably about 0.29 to 4.9 Mpa (3 to 50 kg/cm$^2$)). The amount of the catalyst is not critical, but generally the catalyst is used in an amount corresponding to about 0.1 to 2 times the weight of biphenyl or the mixture of biphenyl and isopropyl-biphenyl.

The reaction may be conducted either batchwise or continuous. The continuous reaction may be conducted in any system such as the fixed bed, fluidized bed, slurry bed, moving bed and so on.

After completion of the reaction according to the process of the invention, the reaction product contains impurities such as unreacted starting materials, catalyst, solvent, mono-and diisopropylbiphenyl and so on. Of these impurities, solid matters such as catalyst can be easily removed by the known solid-liquid separation techniques such as filtration, precipitation and so on. The remaining liquid fraction is then distilled to separate diisopropylbiphenyl from unreacted biphenyl, monoisopropylbiphenyl, solvent and so on. The diisopropylbiphenyl fraction thus separated contains not only 4,4'-diisopropylbiphenyl but also other isomers. Separation of 4,4'-diisopropylbiphenyl can be accomplished by various procedures but it is most advantageous to selectively crystallize out 4,4'-diisopropylbiphenyl under high pressure. More in detail, 4,4'-diisopropylbiphenyl can be isolated in high purity by subjecting the diisopropylbiphenyl fraction to a temperature of about 25 to 120°C (preferably about 40 to 80°C) and a pressure of about 9.8 to 392 Mpa (100 to 4000 kg/cm$^2$) (preferably about 49 to 147 Mpa (500 to 1500 kg/cm$^2$) and, more preferably about 78 to 118 Mpa (800 to 1200 kg/cm$^2$)) to form a solid-liquid mixture and removing the liquid fraction from the mixture.

Effects of the Invention

It remains to be fully elucidated why the present invention provides the described beneficial effects, namely high p-position selectivity, high yield and improved resistance of the catalyst to sulfur poisoning.

However, it is evident, from the results of Comparative Examples described hereinafter, that the above beneficial effects of the invention cannot be achieved when a mordenite type zeolite catalyst with a $SiO_2/Al_2O_3$ molar ratio of less than 15 is employed, when a non-hydrated mordenite type zeolite catalyst is employed, when an alkylating agent other than the one used in the present invention is employed, or when the reaction is conducted in the absence of water.

Thus, when biphenyl with a sulfur content of about 15 ppm is reacted with propylene in a continuous reactor in the presence of a non-hydrated mordenite type zeolite catalyst in accordance with the known process described in Japanese Kokai Patent Application No. 63-122636 or No. 1-165531 referred to hereinbefore, the conversion of biphenyl to diisopropylbiphenyl drops to 5% or less within 100 hours after the start of reaction. However, in the process according to the invention wherein the reaction is conducted using a hydrated mordenite type zeolite catalyst in the presence of water, the conversion is still more than 20% even after 1000 hours of reaction, even when the sulfur content of the starting biphenyl is the same.

Moreover, when the starting biphenyl is one having a sulfur content of about 3000 ppm as available on distillation of coal tar, whereas the conventional process cannot be conducted in a continuous manner, the process of the invention permits almost as many hours of continuous reaction as is the case when biphenyl with a sulfur content of about 15 ppm is employed.

Furthermore, when the resultant diisopropylbiphenyls with high p-selectivity is subjected to high-pressure precipitation, 4,4'-diisopropylbiphenyl is selectively precipitated with a super-high purity not atainable by the conventional processes.

3

Examples

The following examples and comparative examples are given to show the features of the present invention with greater clarity.

Examples 1-2 and Comparative Examples 1-4

(Preparation of catalysts)

(a) A powdery hydrogen ion-exchanged mordenite type zeolite ($SiO_2/Al_2O_3$ molar ratio = 20, 110 or 128) was heated in the air at 450° for 5 hours to give an anhydrous H-mordenite type zeolite.

(b) A powdery hydrogen ion-exchanged mordenite zeolite ($SiO_2/Al_2O_3$ molar ratio = 110 or 128) was stirred in 2% aqueous ammonia at 70 - 80°C for 4 hours, then washed with pure water and dried in the air to give an $NH_4$-mordenite type zeolite. Five parts of $NH_4$-mordenite type zeolite was put in 100 parts of water adjusted to pH 9-10 with aqueous ammonia beforehand and, then, an aqueous solution of [Pt-$(NH_3)_4$]$Cl_2$ was added in such an amount that the amount of Pt supported on zeolite was 0.5%. The mixture was stirred at room temperature for 24 hours, after which the zeolite was washed with water, dried in the air at 150°C for 3 hours, fired in the air at 300°C for 3 hours and finally reduced in a hydrogen gas stream at 450°C for 6 hours to give an anhydrous Pt-mordenite catalyst.

(c) Each of the anhydrous mordenite type zeolites obtained in (a) and (b), 20 grams, was spread thinly on a porcelain which was then set in a horizontal position in a hermetically closable broad-mouthed pressure-resistant glass vessel (capacity 3000 ml) containing 5 ml of water. The vessel was then closed tightly. This pressure-resistant vessel was allowed to stand in thermostat at a constant temperature of 110°C for 12 hours and, then, allowed to cool over 12 hours. The procedure gave a hydrated H-mordenite type zeolite catalyst and a hydrated Pt-mordenite type zeolite catalyst.

[Isopropylation]

A 100 ml autoclave equipped with a rotary stirrier and having a pressure resistance of 9.8 Mpa (100 kg/cm$^2$•G) was charged with 10 g of high-purity biphenyl containing 40 ppm of benzothiophene as sulfur impurity, 20 ml of decaline and 4 g of the hydrated or anhydrous catalyst and the internal pressure was increased to 0,49 Mpa (5 kg/cm$^2$•G) with nitrogen gas. The autoclave was then heated at 250°C. The reaction was conducted with stirring at 250°C for 2 hours while 20 ml of liquefied propylene and a predetermined amount of water was introduced (Examples) or 20 ml of liquefied propylene only was introduced (Comparative Examples).

After completion of the reaction, the catalyst was filtered off and the composition of the reaction mixture was analyzed by gas chromatography. The reaction data were then calculated.

The reaction conditions and results are set forth in Table 1.

Table 1

| | | Example | | Comparative Example | | | |
|---|---|---|---|---|---|---|---|
| | | 1 | 2 | 1 | 2 | 3 | 4 |
| $SiO_2/Al_2O_3$ molar ratio of mordenite catalyst | | 128 | 128 | 128 | 128 | 128 | 128 |
| Platinum on mordenite catalyst | (wt. %) | 0 | 0.5 | 0 | 0.5 | 0 | 0 |
| Degree of hydration of mordenite catalyst | (wt. %) | 20 | 20 | 0 | 0 | 0 | 40 |
| Benzothiophene level in biphenyl | (ppm) | 40 | 40 | 40 | 40 | 40 | 40 |
| Water-biphenyl charge ratio | (wt. %) | 10 | 10 | 0 | 0 | 20 | 0 |
| Conversion of biphenyl | (%) | 97 | 95 | 82 | 63 | 78 | 37 |
| Mono-alkylation | (%) | 14 | 12 | 55 | 50 | 57 | 63 |
| Di-alkylation | (%) | 77 | 83 | 37 | 45 | 36 | 34 |
| 4,4'-selectivity | (%) | 76 | 74 | 68 | 65 | 70 | 71 |
| Yield of 4,4'-diisoproylbiphenyl | (%) | 57 | 58 | 21 | 18 | 20 | 9 |

Table 1 (continued)

(Note) Methods for calculation of reaction data

Conversion of biphenyl = 100 − concentration of biphenyl in reaction product (mol %)

$$\text{Mono-alkylation} = 100 \times \frac{\text{concentration of monoisopropylbiphenyl in reaction product (mol \%)}}{\text{Conversion of biphenyl}}$$

$$\text{Di-alkylation} = 100 \times \frac{\text{concentration of diisopropylbiphenyl in reaction product (mol \%)}}{\text{Conversion of biphenyl}}$$

$$4'4'\text{-selectivity} = 100 \times \frac{\text{concentration of } 4,4'\text{-diisopropylbiphenyl in reaction product (mol \%)}}{\text{Concentration of diisopropylphenyl in reaction product (mol \%)}}$$

$$\text{Yield of } 4,4'\text{-diisopropylbiphenyl} = (\text{Conversion of biphenyl}) \times (\text{dialkylation}) \times (4,4'\text{-selectivity}) \times \frac{1}{10000}$$

The results summarized in Table 1 clearly indicate the excellent effects of the process of the invention in which the reaction is conducted using a hydrated mordenite catalyst in the presence of water.

6

Example 3

A one-liter stainless steel autoclave having a pressure resistance of 4.9 Mpa (50 kg/cm$^2$) and equipped with a stirrer was charged with 300 g of biphenyl containing 40 ppm of benzothiophene and the same hydrated H-mordenite catalyst as used in Example 1 and the temperature was increased to 250°C with constant stirring. Then, 7 liters/hr of propylene and 1.2 g/hr of water were continuously introduced into the gaseous phase in the autoclave. The pressure of the gaseous phase was maintained at 0.39 Mpa (4 kg/cm$^2$) with a pressure control valve, with the excess gas being released via the same valve. A sampling line was provided for sampling the liquid phase during the reaction and samples of the reaction mixture were analyzed with a capillary gas chromatoaraphic analyzer.

The development of reaction with the lapse of time is shown in Table 2.

When compared with Comparative Example 5 described below, the excellent effects of the invention are quite obvious.

Comparative Example 5

Biphenyl was isopropylated by the same procedure as Example 3 except that no water was added during the reaction.

The results are shown in Table 3.

Table 2

| Reaction time (hr) | 2 | 6 | 18 |
|---|---|---|---|
| Conversion (%) | 46 | 82 | 97 |
| Mono-alkylation (%) | 79 | 61 | 43 |
| Di-alkylation (%) | 20 | 38 | 56 |
| 4,4'-selectivity (%) | 71 | 73 | 74 |
| Yield of 4,4'-diisopropylbiphenyl (%) | 7 | 22 | 40 |

Table 3

| Reaction time (hr) | 2 | 6 | 18 |
|---|---|---|---|
| Conversion (%) | 49 | 63 | 64 |
| Mono-alkylation (%) | 74 | 66 | 64 |
| Di-alkylation (%) | 26 | 34 | 36 |
| 4,4'-selectivity (%) | 68 | 69 | 68 |
| Yield of 4,4'-diisopropylbiphenyl (%) | 8 | 15 | 16 |

Reference Examples 1-2

The reaction product of Example 1 was subjected to atmospheric distillation to separate a diisopropylbiphenyl fraction. The content of p,p'-components of the fraction is given in Table 4. The content of p,p'-components is substantially equal to that of the reaction product.

The diisopropylbiphenyl fraction obtained as above was put in a vessel controlled at a constant temperature of 64°C. Then, a pressure of 98 Mpa (1000 kg/cm$^2$•G) was applied and the liquid within the vessel was removed while the pressure was slowly lowered to 19.6 Mpa (200 kg/cm$^2$).

The white crystals of 4,4'-diisopropylphenyl that remained in the vessel were analyzed by gas chromatography. The purity thus found is shown as the result of Reference Example 1 in Table 4.

As a control, the reaction product with a content of p,p'-component of 66% was similarly treated. The results are shown under the Reference Example 2 in Table 4.

7

Table 4

| | Reference Example 1 | Reference Example 2 |
|---|---|---|
| p,p'-Rate of reaction product (%) | 76 | 66 |
| p,p'-Rate of diisopropylbiphenyl fraction (%) | 76 | 66 |
| p,p'-Rate after pressure crystallization (%) | 93 | 84 |
| Yield of p,p'-diisopropylation product in pressure crystallization(%) | 38 | 23 |

It is apparent from Table 4 that, from the highly p,p'-selective diisopropyl fraction obtainable by the process of the invention, 4,4'-diisopropylbiphenyl with a high purity of 93% can be obtained in high purification yield of 38% by one stage of pressure crystallization.

In contrast, when the p,p'-selectivity of the fraction is low, both the purity and yield of 4,4'-diisopropylbiphenyl are low.

It is, thus, clear that when with the highly p,p'-selective diisopropyl fraction of the invention, a high-purity grade of 4,4'-diisopropylbiphenyl can be obtained in high purification yield.

## Claims

1. A process for producing 4,4'-diisopropylbiphenyl comprising by reacting biphenyl or a mixture of biphenyl and isopropylbiphenyl, the sulfur content of which is not more than 1%, with propylene using a hydrated H-mordenite type zeolite catalyst having a $SiO_2/Al_2O_3$ molar ratio of not less than 15 or the corresponding hydrated Pt-mordenite type zeolite catalyst in the presence of water.

2. A process according to Claim 1 wherein said biphenyl or said mixture of biphenyl and isopropyl-biphenyl has a sulfur content of not more than 5000 ppm.

3. A process according to Claim 1 wherein said $SiO_2/Al_2O_3$ molar ratio is in the range of 15 to 250.

4. A process according to Claim 1 wherein the reaction is conducted in the presence of a solvent for dilution.

## Patentansprüche

1. Verfahren zur Herstellung von 4,4'-Diisopropylbiphenyl, das die Umsetzung von Biphenyl oder eines Gemischs von Biphenyl und Isopropylbiphenyl, dessen Schwefelgehalt nicht mehr als 1 % beträgt, mit Propylen unter Verwendung eines hydratisierten Zeolithkatalysators vom H-Mordenit-Typ, der ein Molverhältnis von $SiO_2$ zu $Al_2O_3$ von mindestens 15 aufweist, oder eines entsprechenden hydratisierten Zeolithkatalysators vom Pt-Mordenit-Typ in Gegenwart von Wasser umfaßt.

2. Verfahren nach Anspruch 1, wobei das Biphenyl oder das Gemisch aus Biphenyl und Isopropylbiphenyl einen Schwefelgehalt von nicht mehr als 5000 ppm aufweist.

3. Verfahren nach Anspruch 1, wobei das Molverhältnis von $SiO_2$ zu $Al_2O_3$ im Bereich von 15 bis 250 liegt.

4. Verfahren nach Anspruch 1, wobei die Reaktion in Gegenwart eines als Verdünnungsmittel dienenden Lösungsmittels durchgeführt wird.

## Revendications

1. Procédé de fabrication de 4,4'-diisopropylbiphényle comprenant la réaction du biphényle ou d'un mélange de biphényle et d' isopropylbiphényle, dont la teneur en soufre n' excède pas 1 %, avec du propylène, en utilisant un catalyseur zéolite du type H-mordenite hydraté ayant un ratio molaire $SiO_2/Al_2O_3$ non inférieur à 15 ou le catalyseur zéolite correspondant du type Pt-mordenite, en présence d'eau.

**2.** Procédé suivant la revendication 1, dans lequel ledit biphényle ou ledit mélange de biphényle et d'isopropylbiphényle a une teneur en soufre n'excédant pas 5000 ppm.

**3.** Procédé suivant la revendication 1 dans lequel ledit ratio molaire $SiO_2/Al_2O_3$ est compris dans l'intervalle 15 à 250.

**4.** Procédé suivant la revendication 1, dans lequel la réaction est conduite en présence d' un solvant pour dilution.